# DEMANDE DE BREVET EUROPEEN

(11) **EP 4 059 573 A1**
(43) Date de publication de la demande: **21.09.2022**
(21) Numéro de dépôt: 22163051.0
(22) Date de dépôt: 18.03.2022
(51) Int. Cl.: A61Q 1/14, A61Q 5/00, A61Q 13/00, A61Q 1/00, A61Q 17/04, A61K 8/37, A61K 8/42, A61K 8/60

(54) **AGENTS SOLUBILISANTS ET COMPOSITIONS LES COMPRENANT**

(30) Priorité: 19.03.2021 FR 2102795
(71) Demandeur: B&Sens, 94000 Creteil (FR)
(72) Inventeur: THEVENOT, Laurent, 91480 QUINCY-SOUS-SENART (FR)
(74) Mandataire: Lavoix

(57) **Abrégé**

La présente invention concerne de nouveaux agents solubilisants, en particulier de nouveaux agents solubilisants de phase(s) grasse(s) dans des compositions aqueuses.

La présente invention concerne également de nouvelles compositions aqueuses comprenant lesdits agents solubilisants.

## Description

La présente invention concerne de nouveaux agents solubilisants, en particulier de nouveaux agents solubilisants de phase(s) grasse(s) dans des compositions aqueuses.

La présente invention concerne également de nouvelles compositions aqueuses comprenant lesdits agents solubilisants.

De nombreuses préparations cosmétiques nécessitent la solubilisation de phases grasses dans des phases aqueuses en utilisant des alcools ou tensioactifs.

Du fait de leur caractère lipophile, certains composés sont plus ou moins difficiles à solubiliser dans ces formulations. Ils limitent en outre la concentration en actifs dans ces formules ce qui ne permet pas d'obtenir des produits suffisamment efficaces.

Afin de solubiliser et stabiliser ces composés lipophiles, il est connu d'utiliser des tensioactifs oxyéthylénés issus en partie de la pétrochimie (par exemple l'huile de ricin hydrogénée oxyéthylénée).

Or, les consommatrices sont de plus en plus à la recherche de produits cosmétiques formés, en tout ou partie, de constituants naturels ou d'origine naturelle. Par « naturel », il est entendu conforme au référentiel Européen « COSMOS ».

En particulier, ne sont pas d'origine naturelle: les greffons pétrochimiques, les conservateurs et les agents dénaturants d'origine pétrochimique.

Il subsiste donc le besoin de disposer d'un système solubilisant des composés lipophiles compatible avec la formulation de produits cosmétiques « naturels » et/ou « certifiés biologiques », qui permette la formulation de ces composés lipophiles sans limitation de concentration, de manière à obtenir des formulations stables, efficaces, agréables à l'usage et présentant un aspect esthétique attrayant, notamment un aspect limpide, non trouble.

L'inventeur de la présente invention a mis au point une nouvelle composition qui présente des propriétés de solubilisation plus importantes que les compositions naturelles actuellement disponibles sur le marché, et proche des meilleures solutions contenant des tensioactifs oxyéthylénés.

Ainsi, la présente invention concerne de nouveaux agents solubilisants, en particulier de nouveaux agents solubilisants de phase(s) grasse(s) dans des compositions aqueuses.

L'invention concerne en particulier une composition comprenant :
- au moins un ester d'acide gras de saccharose choisi parmi les esters issus de la réaction de saccharose(s) et d'acide(s) gras comprenant de 10 à 24 atomes de carbone présents en une quantité allant de 1% à 25% en poids par rapport au poids total de la composition ; et
- au moins un N-alkanoyl-N-alkyl glucamide d'acide gras présent en une quantité allant de 10% à 80% en poids par rapport au poids total de la composition.

Dans le cadre de la présente invention, ladite composition mentionnée ci-dessus est appelée composition C1.

En particulier, ladite composition comprend en outre au moins un alkylpolyglucoside présent en une quantité allant de 1% à 70% en poids par rapport au poids total de la composition.

L'invention concerne également une composition aqueuse comprenant :
- une phase aqueuse ;
- la composition C1 susmentionnée; et
- une phase grasse.

Dans le cadre de la présente invention, ladite composition aqueuse est appelée composition C2.

### Ester d'acide gras de saccharose

Par « ester d'acide gras de saccharose » ou « ester de sucrose et d'acide gras », on entend les esters issus de la réaction de sucrose(s) (saccharose(s)) et d'acide(s) gras comprenant de 10 à 24 atomes de carbone, de préférence de 12 à 16 atomes de carbone.

Les acides gras comprenant de 10 à 24 atomes de carbone peuvent être linéaires ou ramifiés, saturés ou insaturés.

En particulier, les acides gras peuvent être choisis parmi l'acide oléique, l'acide laurique, l'acide palmitique, l'acide myristique, l'acide stéarique, l'acide linoléique, l'acide caprique, ou leurs mélanges.

Selon un mode de réalisation, l'ester de saccharose(s) et d'acide(s) gras est choisi parmi les esters issus de la réaction de saccharose(s) et d'acide(s) gras comprenant de 12 à 18 atomes de carbone, de préférence de 12 à 16 atomes de carbone tels que l'acide laurique et/ou l'acide palmitique comme par exemple le sucrose laurate, le sucrose palmitate ou un mélange.

Selon un mode de réalisation, la composition selon l'invention est caractérisée en ce que ledit au moins un ester d'acide gras de saccharose est choisi parmi les esters issus de la réaction de saccharose(s) et d'acide(s) gras comprenant de 12 à 20 atomes de carbone, de préférence de 12 à 18 atomes de carbone et encore plus préférentiellement de 12 à 16 atomes de carbone, et est en particulier choisi parmi le sucrose de laurate, le sucrose de palmitate ou leur mélange.

Les esters de saccharose(s) et d'acide(s) gras selon l'invention peuvent être choisis parmi les mono-, di-, tri- et tétra esters, les polyesters et leurs mélanges.

Des esters à faible degré d'estérification comme par exemple des monoesters, diesters, triesters de saccharose et d'acide gras ou un mélange sont plus particulièrement utilisés.

L'ester de saccharose et d'acide gras peut se présenter sous forme d'un mélange d'esters à faible degré d'estérification comme par exemple un mélange de monoester et diester ou un mélange de monoester, diester et triester.

Un mélange d'esters de saccharose(s) et d'acide(s) gras comprenant de 12 à 16 atomes de carbone, en particulier un mélange de mono, di et triesters d'acide laurique ou d'acide palmitique, ledit mélange pouvant comprendre de façon minoritaire (en une teneur inférieure ou égale à 40% en poids par rapport au poids du mélange d'esters de saccharose et d'acide gras) des esters de saccharose et d'acides gras dans lequel l'acide gras comprend plus de 16 atomes de carbone est plus particulièrement utilisé.

De préférence, l'ester de saccharose(s) et d'acide(s) gras utilisé dans la présente invention présente une HLB supérieure ou égale à 10, de préférence supérieure ou égale à 12.

Par « HLB » ou « Hydrophilic- Lipophilic Balance », on entend le sens bien connu de l'homme du métier, i.e. l'équilibre entre la dimension et la force du groupe hydrophile et la dimension et la force de groupe lipophile de l'agent tensioactif. La valeur HLB selon GRIFFIN est définie dans J. Soc. Cosm. Chem. 1954 (volume 5), pages 249-256.

On peut citer à titre d'exemples d'esters ou de mélanges d'esters de saccharose(s) et d'acide(s) gras selon l'invention les composés suivants:
- les produits vendus sous la dénomination :
   - Sisterna PS750-C (INCl Sucrose Palmitate), de HLB égale à 16, comprenant environ 80% de monoester, le reste du mélange étant composé de di et triesters,
   - Sisterna L70-C (INCl Aqua (and) Sucrose Laurate (and) Alcohol) de HLB égale à 15, comprenant environ 70% de monoester, le reste du mélange étant composé de di et triesters, et
   - Sisterna SP70-C (INCl Sucrose Stearate) de HLB égale à 15, comprenant environ 70% de monoester, le reste du mélange étant composé de di et triesters,
- les produits de la gamme SURPHOPE commercialisés par la société MITSUBISHI CHEMICAL EUROPE :
   - le Surfhope C-1416, présentant une HLB de 16, qui est un sucrose myristate comprenant environ 80% de monoester, le reste du mélange étant composé de di et triesters,
   - le Surfhope C-1216 dont le nom INCl est sucrose laurate, de HLB égale à 16 et comprend de 75 à 90% de monoester, le reste du mélange étant composé de di et triesters,
   - le Surfhope C-1215L dont le nom INCl est sucrose laurate, de HLB égale à 15, comprenant environ 70% de monoesters, le reste du mélange étant composé de di et triesters, et
   - le Surfhope C-1616, présentant une HLB de 16, qui est un mélange d'esters de saccharose et d'acides palmitique et/ou stéarique (nom INCl sucrose palmitate), comprenant 80% de monoester, le reste du mélange étant composé de di et triesters.

Dans le cadre de la présente invention, ledit au moins un ester d'acide gras de saccharose choisi parmi les esters issus de la réaction de saccharose(s) et d'acide(s) gras comprenant de 10 à 24 atomes de carbone est présent en une quantité allant de 1% à 25% en poids par rapport au poids total de la composition C1, en particulier en une quantité allant de 15% à 20%, par exemple 15, 16, 17, 18, 19 ou 20%.

### N-alkanoyl-N-alkyl glucamide d'acide gras

Par « N-alkanoyl-N-alkyl glucamide d'acide gras » ou « « N-alkanoyl-N-alkyl glucamide », on entend le sens commun connu de l'homme du métier.

Les N-alkanoyl-N-alkyl glucamide d'acide gras sont en particulier des agrotensioactifs qui ont des propriétés applicatives et environnementales proches des alkylpolyglucosides et dont la production annuelle avoisine les 40 000 t/an. Les plus connus sont les MEGA-n qui sont les N-methylglucamides. Ils sont utilisés pour leur pouvoir moussant et leur pouvoir émulsionnant dans des détergents. La synergie avec d'autres tensioactifs anioniques tels que des alkylsulfates et des alkyléther sulfates, s'est avérée performante dans le domaine de la cosmétique (shampoing, pâte dentifrice, savon) et des lessives pour blanchissement. De tels amides avec des chaînes en C₁₂-C₁₄ comme adoucissants biodégradables sont utilisés pour le traitement de mouchoirs à papier.

La synthèse des N-alkanoyl-N-methylglucamides se déroule en deux étapes tel qu'illustré ci-dessous :

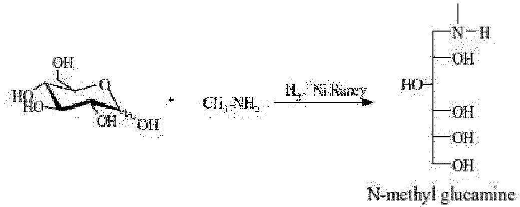

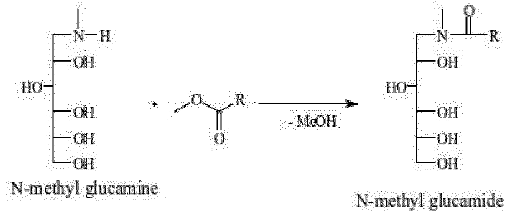

La première étape consiste en une amination réductrice à partir du glucose (ou maltose, lactose, ...) et une amine avec une courte chaîne (C₁-C₄), préférentiellement CH₃NH₂ dans un solvant polaire tel que le méthanol ou l'éthanol à 30-60°C, en présence d'hydrogène et de nickel de Raney ou de palladium sur charbon comme catalyseur. Cette étape peut être aussi réalisée en présence de NaBH₄ ou de cyanoborohydrure de sodium (NaCNBH₃) pour réduire l'imine formée. Par la suite, la N-methylglucamine obtenue peut réagir avec des esters gras (C₆-C₂₀) en présence de bases tels que MeONa, K₂CO₃ ou la pyridine à 40-100°C. L'amidation peut aussi être réalisée avec des chlorures d'acide comme décrite par Wilk et coll. pour la préparation de N-alkanoyl-N-methyl lactitolamine ou en présence d'enzyme (Candida Antarctica lipase) à partir des acides gras tels que l'acide oléique.

Les N-alkanoyl-N-alkyl glucamides pouvant être utilisés dans la composition C1 selon l'invention sont en particulier celles ayant des chaines N-alkanoyl en C₆-C₂₀ et plus particulièrement en C₈ - C₁₄ et/ou ayant des chaines N-alkyl en C₁-C₁₀ et plus particulièrement en C₁-C₄, de préférence la chaine N-alkyl est représentée par un N-métyl, tels que par exemple :
- le N-Coco-acyl-N-methyl-glucamine;
- le N-C_{8/10}-acyl-N-methyl-glucamine;
- le N-C_{12/18}-acyl-N-methyl-glucamine;
- le N-Lauryl-N-Methyl glucamide ;
- le Sunfloweroyl Methylglucamide ;
- le Nonanoyl-N-Methylglucamide; et
- le Decanoyl-N-Methylglucamide.

Encore plus particulièrement, les exemples suivants peuvent être cités :
- les produits de la gamme Glucotain^{®} de la société CLARIANT :
   - N-Coco-acyl-N-methyl-glucamine (INCl Cocoyl Methyl Glucamide), par exemple vendu sous la dénomination GlucoTain^{®} Care ;
   - N-Sunflower-acyl-N-methyl-glucamine (INCl Name: Sunfloweroyl Methylglucamide), par exemple vendu sous la dénomination GLUCOTAIN^{®} SENSE ;N-C_{8/10}-acyl-N-methyl-glucamine (INCl Capryloyl/Caproyl Methyl Glucamide), par exemple vendu sous la dénomination GlucoTain^{®} Clear ; et
   - N-C_{12/18}-acyl-N-methyl-glucamine (INCl Lauroyl/Myristoyl Methyl Glucamide), par exemple vendu sous la dénomination GlucoTain^{®} Flex.
- les produits suivants :
   - Nonanoyl-N-Methylglucamide, par exemple vendu sous la dénomination MEGA-9 par Cayman Chemicals,; et
   - Decanoyl-N-Methylglucamide, par exemple vendu sous la dénomination MEGA-10, ANAGRADE par G Biosciences.

Dans le cadre de la présente invention, ledit au moins un N-alkanoyl-N-alkyl glucamide d'acide gras est présent en une quantité allant de 10% à 80% en en poids par rapport au poids total de la composition C1, en particulier en une quantité allant de 50% à 75%, par exemple 55, 60, 63, 65, 70, 72 ou 74%.

### Alkylpolyglucoside

Tel que susmentionné, la composition C1 selon l'invention peut comprendre en outre au moins un alkylpolyglucoside présent en une quantité allant de 1% à 70% en poids par rapport au poids total de la composition.

Par « alkylpolyglucoside » ou « APG », on entend le sens commun pour l'homme du métier i.e une classe de tensioactifs non ioniques. Biodégradables et d'origine végétale à partir de sucres, ces tensioactifs sont généralement des dérivés du glucose et des alcools gras. Les matières premières sont généralement de l'amidon et de la graisse, et les produits finaux sont généralement des mélanges complexes de composés avec différents sucres comprenant l'extrémité hydrophile et des groupes alkyle de longueur variable comprenant l'extrémité hydrophobe. Lorsqu'ils sont dérivés du glucose, ils sont connus sous le nom d'alkyl polyglucosides.

Les alkylpolyglucosides sont largement utilisés dans le domaine cosmétique.En particulier, les alkylpolyglucosides sont des mélanges d'alkylpolyglucosides ayant différentes longueurs de chaîne alkyle, et/ou ayant divers degrés de polymérisation, à savoir diverses unités de glucose (ou dérivé de glucose).

Selon l'invention, la composition C1 comprend au moins un alkylpolyglucoside ou un mélange d'alkylpolyglucosides.

Tel que susmentionné, typiquement, les alkylpolyglucosides résultent de la réaction de condensation d'alcool(s) gras et de glucose ou de l'un de ses dérivés.

Dans un mode de réalisation, les alkylpolyglucosides sont préparés à partir d'un mélange d'alcools gras de différentes longueurs de chaîne alkyle, en particulier chaque chaîne alkyle comprenant de 2 à 22 atomes de carbone, de préférence de 8 à 18 atomes de carbone.

En particulier, les alkylpolyglucosides sont des composés de formules (I) :

Rₐ(O)(G)ₓ (I)

dans laquelle:
- Ra représente un groupe alkyle linéaire ou ramifié comprenant de 2 à 22 atomes de carbone;
- G représente un fragment sucre comprenant 5 ou 6 atomes de carbone; et
- x est un entier compris entre 1 et 15, de préférence entre 1 et 10.

Dans un mode de réalisation, les alkylpolyglucosides préférés sont ceux dans lesquels Ra consiste:
- essentiellement de groupes alkyles en C₈ et C₁₀;
- essentiellement de groupes alkyles en C₁₂ et C₁₄;
- essentiellement de groupes alkyles en C₈ à C₁₆; ou
- essentiellement de groupes alkyles en C₁₂ à C₁₆.

Dans un mode de réalisation, dans la formule (I) mentionnée ci-dessus, G représente un fragment glucose, fructose ou galactose. En particulier, G représente un fragment glucose.

Dans un mode de réalisation, Ra peut être un mélange de groupes alkyles linéaires ou ramifiés comprenant de 2 à 22 atomes de carbone. Par exemple, l'alkylpolyglucoside de formule (I) peut être un polyglucoside dans lequel Ra est un octyle et /ou un décyle.

Dans un mode de réalisation, dans la formule (I), Ra est un groupe alkyle linéaire comprenant de 8 à 10 atomes de carbone.

Dans un mode de réalisation, dans la formule (I), x est un entier compris entre 1 et 2, et de préférence entre 1,1 et 1,5.

Dans un mode de réalisation, dans la formule (I), Ra est un groupe alkyle linéaire ou ramifié comprenant de 8 à 18 atomes de carbone, de préférence de 8 à 10 atomes de carbone, G est un fragment glucose et x est un entier compris entre 1 et 2.

Dans un mode de réalisation, l'alkylpolyglucoside (APG) répond à la formule (I') suivante:

Rₐ(O)(G)ₓ (I')

dans laquelle Ra est un mélange de groupes alkyles linéaires ou ramifiés comprenant de 2 à 22 atomes de carbone.

Dans un mode de réalisation, la composition C1 comprend un mélange d'au moins deux composés différents de formule (I).

Par exemple, la composition C1 comprend un mélange d'au moins deux composés différents de formule (I), qui diffèrent par la nature de Ra et/ou la valeur de x.

Dans un mode de réalisation, la composition C1 comprend un mélange de:
- un composé de formule (I) dans laquelle Ra est un groupe alkyle linéaire comprenant 8 atomes de carbone; et
- un composé de formule (I) dans laquelle Ra est un groupe alkyle linéaire comprenant 10 atomes de carbone.

En particulier, l'alkylpolyglucoside est choisi dans le groupe constitué par: (C₁₂-C₂₀)alkylpolyglucoside, (C₈-C₁₆)alkylpolyglucoside, (C₈-C₁₀)alkylpolyglucoside, (C₁₂-C₁₆)alkylpolyglucoside, (C₁₂-C₁₄)alkylpolyglucoside, (C₁₂-C₁₄)alkylpolyglucoside, leurs mélanges.

Plus particulièrement, l'alkylpolyglucoside est choisi dans le groupe constitué de: cocoglucoside (INCl), décyl glucoside (INCl), lauryl glucoside (INCl), caprylyl/capryl glucoside (INCl), arachidyl glucoside (INCl), butyl glucoside (INCl), caprylyl glucoside (INCl), cetearyl glucoside (INCl), hexadécyl Dglucoside (INCl), isostéaryl glucoside (INCl), éthyl glucoside (INCl), myristyl glucoside (INCl), octadécyl D-glucoside (INCl), octyldodécyl glucoside (INCl), undécyl glucoside (INCl), et leurs mélanges.

En particulier, comme cela est connu dans le domaine cosmétique, les noms INCl mentionnés ci-dessus de l'alkylpolyglucoside recouvrent un mélange de glucosides d'alcools gras ayant différentes longueurs de chaîne alkyle et/ou degrés de polymérisation.

Par exemple, le lauryl glucoside est un (C₁₂-C₁₆) alkylpolyglucoside.

Encore plus particulièrement, les alkylpolyglucosides selon l'invention sont choisis parmi :
- le cocoglucoside (INCl) commercialisé sous la dénomination commerciale Plantacare 818^{®} de BASF ou BergaSoft CG 50/MB de Berg + Schmidt ou Sucranov 818UP de Jarchem ;
- le décyl glucoside (INCl) commercialisé sous la dénomination commerciale Plantacare 2000^{®} UP de BASF ou BergaSoft CG 50/MB de Berg + Schmidt ou Sucranov 818 UP de Jarchem ;
- le lauryl glucoside (INCl) commercialisé sous le nom commercial Plantacare 1200^{®} de BASF ou BergaSoft LG 50/MB de Berg + Schmidt ou Sucranov 1200 UP de Jarchem ; et
- le caprylyl/capryl glucoside (INCl) commercialisé sous la dénomination commerciale BergaSoft CCG 70/MB de Berg + Schmidt, Sucranov 810 UP de Jarchem et leurs mélanges.

Dans le cadre de la présente invention, ledit au moins un alkylpolyglucoside est présent en une quantité allant de 1% à 70% en en poids par rapport au poids total de la composition C1, en particulier en une quantité allant de 1% à 10%, par exemple 2, 3, 4, 5, 6, 7, 8 ou 9%.

### Autres ingrédients

La composition C1 selon l'invention peut comprendre en outre de la glycérine.

En particulier, la composition C1 selon l'invention comprend en outre entre 0 et 35% de glycérine, de préférence entre 0 et 10%, en poids par rapport au poids total de la composition C1, par exemple entre 1 et 35%, de préférence par exemple entre 1 et 10%.

Ainsi la composition C1 selon l'invention comprend par exemple 2, 3, 4, 5, 6, 7, 8 ou 9% de glycérine, en poids par rapport au poids total de la composition.

Plus particulièrement, la glycérine utilisée dans le cadre de la présente invention est d'origine végétale.

### Préparation de la composition C1 selon l'invention

La présente invention concerne également le procédé de préparation de la composition C1 selon l'invention, comprenant le mélange successif à température ambiante du N-alkanoyl-N-alkyl glucamide, de l'APG et éventuellement de la glycérine si elle est présente. L'ester d'acide gras de saccharose est ensuite ajouté.

Le mélange sous agitation est chauffé pendant une certaine période (par exemple 20 minutes à 75°C).

L'agitation est ensuite coupée.

En cas de présence de mousse, la formulation est versée dans un récipient adapté en évitant à la mousse de se déverser.

Le reste de composition et de mousse est ensuite remis à chauffer sous agitation lente (par exemple pendant 5 minutes) puis versé dans le récipient une fois le reste de mousse éliminé.

La composition C1 selon l'invention comprend par ailleurs avantageusement des matières premières renouvelables.

### Utilisation de la composition C1 selon l'invention

La présente invention concerne également l'utilisation de la composition C1 précitée en tant qu'agent solubilisant.

Tel qu'utilisé ici, le terme « agent solubilisant » ou « solubilisant » désigne un agent qui permet la solubilisation d'une phase grasse liquide ou solide dans un milieu aqueux, en particulier dans une composition aqueuse.

L'inventeur a constaté que l'agent solubilisant permet avantageusement l'obtention de compositions aqueuses transparentes en solubilisant une phase grasse dans un milieu aqueux. Ceci est particulièrement intéressant étant donné que les compositions cosmétiques transparentes sont particulièrement adaptées aux clients. Tel qu'utilisé ici, le terme « composition transparente » désigne une composition transparente à l'œil nu.

### Compositions cosmétiques

La présente invention concerne également une composition aqueuse C2 comprenant:
- une phase aqueuse;
- la composition C1 telle que définie ci-dessus; et
- une phase grasse.

Dans un mode de réalisation, la phase grasse comprend au moins un composé choisi dans le groupe constitué par: les agents colorants, les substances odorantes d'origine synthétique, les composés aromatiques parfumés, les acides gras, les esters d'acides gras, les alcools gras, les vitamines, les extraits naturels, les huiles, les parfums, filtres UV et leurs mélanges.

Dans un mode de réalisation, les alcools gras, saturés ou insaturés, linéaires ou ramifiés, comportent de 8 à 26 atomes de carbone, sont notamment choisis dans le groupe constitué par l'alcool cétylique, l'alcool stéarylique, l'octyldodécanol, le 2-butyloctanol, le 2-hexyldécanol, le 2-undécylpentadécanol, l'alcool oléylique, l'alcool linoléique, le pentaérythrytol, l'alcool laurique, l'alcool benzylique, l'alcool isostéarylique, l'alcool béhénylique, l'alcool cétéarylique, l'alcool isocétylique, l'alcool myristylique, l'alcool de lanoline et leurs mélanges.

Dans un mode de réalisation, les esters d'acides gras sont des esters d'acides gras et d'alcools gras. En particulier, l'acide gras est un acide carboxylique linéaire ou ramifié, saturé ou insaturé, comprenant de 4 à 36 atomes de carbone, de préférence 4 à 22 atomes de carbone, plus préférentiellement 6 à 12 atomes de carbone, en particulier 7 à 10 atomes de carbone.

Dans un mode de réalisation, l'acide gras est choisi dans le groupe constitué par: l'acide caprylique, l'acide caprique, l'acide laurique, l'acide myristique, l'acide palmitique, l'acide stéarique, l'acide arachidique, l'acide béhénique, l'acide lignocérique, l'acide cérotique et leurs mélanges.

Dans un mode de réalisation, les esters d'acides gras sont le cocoate de méthyle/ ricinoléate/isostéarate, le cétéaryloctanoate, le pentaérythrylisostéarate ou le nézoate de benzyle.

Dans un mode de réalisation, les substances odorantes d'origine synthétique sont des composés comprenant au moins l'un du groupe choisi parmi les groupes ester, éther, aldéhyde, cétone, alcool aromatique et hydrocarbure.

Par exemple, comme esters, on peut citer l'acétate de benzyle, le benzoate de benzyle, l'isobutyrate de phénoxyéthyle, l'acétate de p-tert-butylcyclohexyle, l'acétate de citronellyle, le formiate de citronellyle, l'acétate de géranyle, l'acétate de linalyle, l'acétate de diméthylbenzylcarbonyle, l'acétate de phényle formate, le benzoate de benzyle, le glycinate d'éthylméthylphényle, le propionate d'alkylcyclohexyle, le propionate de styralyle, le salicylate de benzyle, l'acétate d'éthyle, le benzoate d'éthyle, le butyrate d'éthyle, l'hexanoate d'éthyle, le cinnamate d'éthyle, le formate d'éthyle, l'heptanoate d'éthyle, l'isovalérate d'éthyle, le lactate d'éthyle, le nonanoate d'éthyle, le pentanoate d'éthyle, l'acétate de géranyle butyrate, le pentanoate de géranyle, l'acétate d'isobutyle, le formate isobutyle, l'acétate d'isoamyle, l'acétate d'isopropyle, l'acétate de linalyle, le butyrate de linalyle, le formate de linalyle, l'acétate de méthyle, l'anthranilate de méthyle, le benzoate de méthyle, le butyrate de méthyle, le cinnamate de méthyle, le salpentanoate, le méthylphénicacétate de méthyle, l'acétate d'octyle, le butyrate d'octyle, l'acétate d'amyle, le butyrate de pentyle, l'hexanoaz de pentyle, le pentanoate de pentyle, l'acétate de propyle, l'hexanoate de propyle, l'isobutyrate de propyle et le butyrate de terpényle.

Par exemple, comme éthers, on peut citer l'éther benzyléthylique.

Par exemple, comme aldéhydes, on peut citer les alcanals linéaires comprenant de 8 à 18 atomes de carbone, le citral, le citronellal, le citronellyloxyacétaldéhyde, le cyclamen aldéhyde, l'hydroxycitronellal, le lilial et le bourgeonal.

Par exemple, comme cétones, on peut citer les ionones, par exemple l'α-isométhylionone, et la méthyl cédryl cétone.

Par exemple, comme alcools aromatiques, on peut citer les alcools terpéniques, tels que l'anéthol, le citronellol, l'eugénol, l'isoeugénol, le géraniol, le linalol, l'alcool phényléthylique et le terpinéol.

Dans un mode de réalisation, l'huile est choisie dans le groupe constitué par: les huiles minérales, les huiles synthétiques, les huiles essentielles, les huiles végétales et leurs mélanges.

Dans un mode de réalisation, les huiles essentielles sont choisies dans le groupe constitué par: l'huile d'anis; l'huile de basilic; l'huile de bergamote; l'huile d'amande amère; l'huile de camphre; l'huile d'agrumes; l'huile de citron; l'huile d'eucalyptus; l'huile de géranium; l'huile de pamplemousse; l'huile de gingembre; l'huile de camomille; l'huile de menthe verte, l'huile de carvi, l'huile de lime; l'huile de mandarine; l'huile de clou de girofle (fleur), l'huile d'orange; l'huile de menthe poivrée; l'huile de rose; l'huile de romarin; l'huile de sauge; l'huile d'achillée millefeuille; l'huile d'anis étoilé; l'huile de thym; l'extrait de vanille; l'huile de baies de genièvre; l'huile de thé des bois; l'huile de feuille de cannelle; l'huile d'écorce de cannelle; l'huile de lavandin et leurs mélanges.

Dans un mode de réalisation, les huiles végétales sont choisies dans le groupe constitué par: l'huile d'amande, l'huile de noyau d'abricot, l'huile de jojoba, l'huile d'argan, le beurre de cisaillement, l'huile de pépins de raisin, l'huile d'avocat, l'huile de macadamia, l'huile de tournesol et leur mélange.

Dans un mode de réalisation, les huiles minérales sont choisies dans le groupe constitué par l'isohexadécane, la cera microcristallina, la cérésine, le polyisobutène hydrogéné, l'isododécane, l'ozokérite, le paraffinum liquidum, les alcanes, la paraffine, la vaseline et leurs mélanges.

Dans un mode de réalisation, les huiles synthétiques sont choisies dans le groupe constitué par les silicones et la cire synthétique.

Dans un mode de réalisation, les agents colorants sont choisis dans le groupe constitué de: lycopène, paprika, anthocyane, chlorophylle, curcuma, bêta-carotène, rocou, pigments naturels et synthétiques et leurs mélanges.

Tel qu'utilisé ici, le terme « agent colorant » signifie un agent apportant une couleur à la composition dans laquelle ledit agent est incorporé.

Dans un mode de réalisation, les composés aromatiques parfumés ou les parfums sont des mélanges naturels et/ou synthétiques d'huiles essentielles et de substances odorantes d'origine synthétique.

Dans un mode de réalisation, la composition aqueuse C2 telle que définie ci-dessus comprend de 0,5% à 30%, de préférence de 1% à 25%, et plus préférentiellement de 1,2% à 22% en poids de la composition C1 telle que définie ci-dessus, par rapport au total poids de ladite composition aqueuse C2.

Dans un mode de réalisation, la composition aqueuse C2 comprend de 0,01% à 40%, de préférence de 0,05% à 20%, et plus préférentiellement de 1% à 5% en poids de la phase grasse par rapport au poids total de ladite composition aqueuse C2.

La composition aqueuse C2 de la présente invention comprend typiquement de l'eau en des quantités allant d'environ 50% à 99%, de préférence d'environ 60% à environ 98%, et plus préférablement d'environ 70% à environ 97% en poids sur la base du poids total de la composition.

Dans un mode de réalisation, la composition C2 est une émulsion.

Dans un mode de réalisation, la composition aqueuse C2 est une composition cosmétique.

Dans un mode de réalisation, la composition aqueuse C2 est choisie dans le groupe constitué par: u, parfum, une eau de toilette, un déodorant transparent, une lotion hydratante, un spray hydratant, une eau micellaire, une lotion nettoyante, un gel de solution nettoyante, une lotion nettoyante biphasique, un gel exfoliant nettoyant, un démaquillant tel qu'un démaquillant à rincer ou un démaquillant sans rinçage, un après-rasage sans alcool, une eau purifiante avec des huiles essentielles, un système en phase D, un après-shampooing à pulvériser naturel, une solution pour lingettes humides, un tonique naturel pour le corps, un gel coiffant, une préparation de lotion antipelliculaire, un écran solaire anti-UV, une préparation pour le bain, une préparation pour le soin des cheveux, ou un produit de soin de la peau pour préparation au maquillage, un produit de soin solaire, un produit de soin des cheveux, un produit de maquillage, ou un produit de soin de la peau.

Dans un mode de réalisation, la composition aqueuse C2 peut également comprendre en outre tout additif habituellement utilisé dans le domaine concerné, par exemple choisi parmi les gommes, les résines, les dispersants, les polymères semi-cristallins, les antioxydants, les conservateurs, les neutralisants, les agents antiseptiques, les agents de protection UV, les cosmétiques, des actifs, tels que des vitamines, des agents hydratants, des émollients ou des agents protecteurs de collagène, et leurs mélanges.

L'ajustement de la nature et de la quantité des additifs présents dans les compositions selon l'invention relève des opérations de routine de l'homme du métier, de sorte que les propriétés cosmétiques et les propriétés de stabilité recherchées de ces dernières ne sont pas affectées.

Dans toute la description ci-dessus, sauf indication contraire, le terme « compris entre x et y » ou « allant de x à y » correspond à une plage inclusive, c'est-à-dire que les valeurs x et y sont comprises dans la plage.

Par « au moins un », il est entendu un ou plusieurs, par exemple 1, 2, 3, 4, 5, 6, etc.

Toutes les quantités sont données en pourcentage en poids par rapport au poids total de la composition.

Les noms des composés sont indiqués selon les cas en noms chimiques ou en noms INCl.

Les exemples suivants sont donnés à titre d'illustration de l'invention.

### Exemples

### Préparation d'une composition selon l'invention

Dans un bécher sous agitation, le N-alkanoyl-N-alkyl glucamide, et éventuellement l'APG et la glycérine sont ajoutés successivement. L'ester d'acide gras de saccharose est ensuite ajouté lentement de manière à bien incorporer la poudre. Le mélange sous agitation est porté à 75°C pendant 20 minutes. L'agitation est ensuite coupée et la formulation est ensuite versée dans un récipient adapté en évitant à la mousse de se déverser. Le reste de composition et de mousse est ensuite remis à chauffer sous agitation lente pendant 5 minutes puis versé dans le récipient une fois le reste de mousse éliminé.

### Exemple d'une composition C1 selon l'invention (composition A)

La composition A selon l'invention suivante est préparée selon le procédé susmentionné :

| | |
|---|---|
| - Caprylyl/Capryl Glucoside (UNIVAR): | 7% |
| - Capryloyl/Caproyl Methyl Glucamide (CLARIANT): | 70% |
| - Sucrose Palmitate (MITSUBISHI) : | 18% |
| - Glycerine (AMI CHIMIE) : | 5% |

### Exemple d'une autre composition C1 selon l'invention (composition B) :

La composition B selon l'invention suivante est préparée selon le procédé susmentionné :

| | |
|---|---|
| - Capryloyl/Caproyl Methyl Glucamide (CLARIANT): | 80% |
| - Sucrose Palmitate (MITSUBISHI) : | 20% |

### Performance d'une composition C1 selon l'invention

La performance des compositions A et B susmentionnées a été testée sur la solubilisation de 3 produits lipophiles utilisés habituellement en cosmétique pour leurs propriétés parfumantes :
- Concentré de parfum « GLAMOROUS » (Creascents) ;
- Linalool 95% (TCI Chemicals) ; et
- Huile Essentielle de lavande vraie (Creascents).

La performance a été comparée à différentes autres molécules et compositions également utilisées pour leurs propriétés solubilisantes :
- SOLUB&SENS : solubilisant de haute performance basé sur une association de tensioactifs éthoxylés ;
- Résassol Ultimate : solubilisant naturel basé sur une association d'ester de polyglycerol et d'APG ;
- Makigreen LCS+: Solubilisant naturel basé sur une association de tensioactif gemini, d'ester de polyglycerol et d'APG ;
- Sisterna L70-C : Ester de sucrose : Sucrose Laurate ;
- NIKKOL Decaglyn : 1-L ester de polyglycerol :Polyglyceryl-10 Laurate ; et
- SYNETH C15 LONZA : ester de polyglycerol : Polyglyceryl-10 Caprylate/Caprate.

### Solubilisation de 1g de composés lipophiles dans 8g d'eau distillée :

Les compositions A et B sont mélangées au composé lipophile, puis l'eau est ajoutée jusqu'à ce que la solution soit limpide.

Quantité de produit (g) nécessaire pour que la solution soit limpide :

**[Tableau 1]**

| Solubilisant | Parfum Sucré/Fleuri « Glamorous » | Linalool | H.E. Lavande |
|---|---|---|---|
| Resassol Ultimate | 4,07 | 3,69 | 2,24 |
| SOLUB&SENS | 1,14 | 1,51 | 1,84 |
| **Composition A selon l'invention** | **1,32** | **1,76** | **1,88** |
| **Composition B selon l'invention** | **1,45** | **1,94** | **1,66** |
| MakiGreen LCS+ | 3,27 | 3,6 | 2,27 |
| Sisterna L70-C Sucrose Laurate | 16,6 | 35,7 | - |
| NIKKOL Decaglyn 1-L Polyglyceryl-10 Laurate | 11,47 | - | - |
| SYNETH L15 LONZA | 12,47 | 16 | 16 |

| | | | |
|---|---|---|---|
| - : non testé. | | | |

Les compositions A et B selon l'invention montrent des propriétés de solubilisation proches du solubilisant basé sur les tensioactifs éthoxylés.

Les compositions A et B selon l'invention montrent des propriétés de solubilisation nettement plus importantes que les molécules naturelles et associations de tensioactifs naturels testées.

## Revendications

1. Composition comprenant :
- au moins un ester d'acide gras de saccharose choisi parmi les esters issus de la réaction de saccharose(s) et d'acide(s) gras comprenant de 10 à 24 atomes de carbone présents en une quantité allant de 1% à 25%, de préférence de 15% à 20% en poids par rapport au poids total de la composition ; et
- au moins un N-alkanoyl-N-alkyl glucamide d'acide gras présent en une quantité allant de 10% à 80%, de préférence de 50% à 75% en poids par rapport au poids total de la composition.

2. Composition selon la revendication 1, comprenant en outre entre au moins un alkylpolyglucoside présent en une quantité allant de 1% à 70%, de préférence de 1% à 10% en poids par rapport au poids total de la composition.

3. Composition selon la revendication 1 ou 2, comprenant en outre entre 0 et 35% de glycérine, de préférence entre 0 et 10%, en poids par rapport au poids total de la composition.

4. Composition selon l'une quelconque des revendications précédentes, **caractérisée en ce que** ledit au moins un ester d'acide gras de saccharose est choisi parmi les esters issus de la réaction de saccharose(s) et d'acide(s) gras comprenant de 12 à 20 atomes de carbone, de préférence de 12 à 18 atomes de carbone et encore plus préférentiellement de 12 à 16 atomes de carbone, et est en particulier choisi parmi le sucrose de laurate, le sucrose de palmitate ou leur mélange.

5. Composition selon l'une quelconque des revendications précédentes, **caractérisée en ce que** ledit au moins un N-alkanoyl-N-alkyl glucamide d'acide gras est choisi parmi les un N-alkanoyl-N-alkyl glucamides ayant des chaines N-alkanoyl en C₆-C₂₀, préférentiellement en C₈ - C₁₄ et/ou ayant des chaines N-alkyl en C₁-C₁₀ et plus particulièrement en C₁-C₄.

6. Composition selon l'une quelconque des revendications précédentes, **caractérisée en ce que** ledit au moins un N-alkanoyl-N-alkyl glucamide d'acide gras est choisi parmi :
- le N-Coco-acyl-N-methyl-glucamine;
- le N-C_{8/10}-acyl-N-methyl-glucamine;
- le Sunfloweroyl Methylglucamide ;
- le N-C_{12/18}-acyl-N-methyl-glucamine;
- le N-Lauryl-N-Methyl glucamide ;
- le Nonanoyl-N-Methylglucamide; et
- le Decanoyl-N-Methylglucamide.

7. Composition selon l'une quelconque des revendications 2 à 6, dans laquelle ledit au moins un alkylpolyglucoside répond à la formule (I) suivante :
Ra(O)(G)x (I)
dans laquelle :
- Ra représente un groupe alkyle linéaire ou ramifié comprenant de 2 à 22 atomes de carbone ;
- G représente un fragment sucre comprenant 5 ou 6 atomes de carbone ; et
- X est un entier compris entre 1 et 15.

8. Composition selon l'une quelconque des revendications 2 à 7, dans laquelle le dit au moins un alkylpolyglucoside est choisi dans l'ensemble constitué par : cocoglucoside, décyl glucoside, lauryl glucoside, caprylyl/capryl glucoside, arachidyl glucoside, butyl glucoside, caprylyl glucoside, cetearyl glucoside, éthylglucoside, hexadécylDglucoside, isostéaryl glucoside, myristylglucoside (INCl), octadécyID-glucoside, octyldodécyl glucoside, undécyl glucoside, et leurs mélanges.

9. Composition aqueuse comprenant :
- une phase aqueuse ;
- la composition selon l'une quelconque des revendications 1 à 8 ; et
- une phase grasse.

10. Composition aqueuse selon l'une quelconque des revendications 8 ou 9, qui est un parfum, une eau de toilette, un déodorant transparent, une lotion hydratante, un spray hydratant, une eau micellaire, une lotion nettoyante, un gel de solution nettoyante, une lotion nettoyante biphasique, un gel exfoliant nettoyant, un démaquillant tel qu'un démaquillant à rincer ou un démaquillant sans rinçage, un après-rasage sans alcool, une eau purifiante avec des huiles essentielles, un système en phase D, un après-shampooing à pulvériser naturel, une solution pour lingettes humides, un tonique naturel pour le corps, un gel coiffant, une préparation de lotion antipelliculaire, un écran solaire anti-UV, une préparation pour le bain, une préparation pour le soin des cheveux, ou un produit de soin de la peau pour préparation au maquillage, un produit de soin solaire, un produit de soin des cheveux, un produit de maquillage, ou un produit de soin de la peau
